# EUROPEAN PATENT SPECIFICATION

(11) **EP 2 585 027 B1**
(45) Date of publication and mention of the grant of the patent: **08.11.2017**
(21) Application number: 10728097.6
(22) Date of filing: 23.06.2010
(51) Int. Cl.: A61K 8/11, A61K 8/21, A61K 8/365, A61K 8/44, A61K 8/49, A61K 8/60, A61K 8/73, A61Q 11/00

(54) **ENCAPSULATION OF INGREDIENTS IN LACTOSE MATRIX TO FORM ACTIVE ENCAPSULATES**
VERKAPSELUNG VON INHALTSSTOFFEN IN LACTOSE MATRIX ZUR FORMIERUNG AKTIVER KAPSELN
L'ENCAPSULATION DES INGRÉDIENTS DANS UNE MATRICE DE LACTOSE POUR FORMER DES ENCAPSULÉS ACTIFS

(43) Date of publication of application: 01.05.2013
(73) Proprietor: Colgate-Palmolive Company, New York, NY 10022 (US)
(72) Inventor: FRUGE, Linh, Hillsborough NJ 08844 (US); HEPLER, Barbara, South Bound Brook NJ 08880 (US); COLLINS, Michael, Hazlet NJ 07730 (US); BOYD, Thomas, Metuchen NJ 08840 (US)
(74) Representative: Wibbelmann, Jobst
(86) International application number: PCT/US2010/039673
(87) International publication number: WO 2011/162755

(56) References cited:
- WO-A1-01/07000
- DE-A1- 10 130 128
- DE-A1-102005 057 292
- JP-A- H08 225 447
- US-A1- 2007 275 064
- US-B1- 6 592 852
- K.A Macritchie ET AL: "The evaluation of the rheological properties of lactose/microcrystalline cellulose and water mixtures by controlled stress rheometry and the relationship to the production of spherical pellets by extrusion/spheronization", European Journal of Pharmaceutical Sciences, vol. 17, no. 1-2, 1 October 2002 (2002-10-01), pages 43-50, XP55114727, ISSN: 0928-0987, DOI: 10.1016/S0928-0987(02)00130-6

## Description

### FIELD

This application relates to oral care compositions, and more particularly to compositions comprising lactose matrix encapsulates containing oral care ingredients. Such compositions include, for example, dentifrices. The description further relates to personal care compositions, and more particularly to compositions comprising lactose matrix encapsulates containing personal care ingredients.

### BACKGROUND

An effective dentifrice composition should provide maintenance and preservation of tooth appearance through the thorough cleaning of teeth and gums, removal of dental stains, and the polishing of teeth.

Thus, there is an ongoing need for new oral care compositions, and methods of their use.

WO01/07000 describes dentifrice compositions.

MacRitchie, et al. (European Journal of Pharmaceutical Sciences, 17 (2002) 43-50) describes the evaluation of the rheological properties of lactose/microcrystalline cellulose and water mixtures by controlled stress rheometry and the relationship to the production of spherical pellets by extrusion/spheronization.

DE10 2005 057 292 describes formulations suitable for the care of skin, nails and hair.

DE101 30 128 describes a pharmaceutical composition together with carrier and excipients.

### BRIEF SUMMARY

The present invention provides a composition comprising an encapsulate entrained in a carrier, wherein the encapsulate comprises: at least one oral care ingredient encapsulated in a matrix, wherein the matrix comprises: lactose monohydrate; and a microcrystalline cellulose, wherein the lactose monohydrate is present in an amount of 50% to 75% by weight of the encapsulate, and wherein the oral care ingredient is zinc citrate in an amount of 0.1% to 50% by weight of the encapsulate.

In another aspect not covered by the claims, the present description provides a method of providing oral health benefits to an oral surface comprising contacting the oral surface with a lactose matrix encapsulate having an active ingredient.

### DETAILED DESCRIPTION

The present invention provides compositions for administration to, or use with, a human or other animal subject. Preferably, specific materials and compositions to be used in this invention are, accordingly, pharmaceutically- or cosmetically- acceptable. As used herein, such a "pharmaceutically acceptable" or "cosmetically acceptable" or "orally acceptable" component is one that is suitable for use with humans and/or animals to provide the desired therapeutic, sensory, decorative, or cosmetic benefit without undue adverse side effects (such as toxicity, astringent taste, irritation, and allergic response) commensurate with a reasonable benefit/risk ratio. The following definitions and non-limiting guidelines should be considered in reading and interpreting the description of this invention set forth herein.

As used herein, the words "preferred" and "preferably" refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention. As used herein, the word "include," and its variants, is intended to be non-limiting, such that recitation of items in a list is not to the exclusion of other like items that may also be useful in the materials, compositions, devices, and methods of this invention. In a similar manner, the description of certain advantages or disadvantages of known materials and methods is not intended to limit the scope of the embodiments to their exclusion. Indeed, certain embodiments may include one or more known materials or methods, without suffering from the disadvantages discussed herein.

The expression "effective amount," as used herein denotes an amount of a compound or composition sufficient to significantly induce a positive benefit, preferably an oral health benefit, but low enough to avoid serious side effects, i.e., to provide a reasonable benefit to risk ratio, within the sound judgment of a person having ordinary skill in the art. The use of singular identifiers such as "the," "a," or "an" is not intended to be limiting solely to the use of a single component, but may include multiple components.

An oral care composition of the present invention can take any form suitable for application to an oral surface. In various illustrative embodiments the composition can be a liquid solution suitable for irrigating, rinsing or spraying; a dentifrice such as a powder, toothpaste or dental gel; a periodontal gel; a liquid suitable for painting a dental surface (*e.g.,* a liquid whitener); a chewing gum; a dissolvable, partially dissolvable or non-dissolvable film or strip (*e.g.,* a whitening strip); an encapsulate (e.g., composition encapsulated in gelatin), a wafer; a lozenge, a wipe or towelette; an implant; a mouthrinse, a foam, a dental floss; *etc.* The composition can contain active and/or carrier ingredients additional to those recited above.

The expressions "carrier" or "aqueous carrier" as used throughout this description denote any safe and effective carrier materials for use herein, such as water, solvents, oils, gels, and the like. Such carriers may include, for example, thickening agents, humectants, ionic active ingredients, buffering agents, anticalculus agents, abrasive polishing materials, peroxide sources, alkali metal bicarbonate salts, surfactants, titanium dioxide, coloring agents, flavor systems, sweetening agents, antimicrobial agents, herbal agents, desensitizing agents, stain reducing agents, and mixtures thereof.

Unless otherwise specified, all percentages and amounts expressed herein and elsewhere in the specification should be understood to refer to percentages by weight. The amounts given are based on the active weight of the material. The recitation of a specific value herein is intended to denote that value, plus or minus a degree of variability to account for errors in measurements. For example, an amount of 10% may include 9.5% or 10.5%, given the degree of error in measurement that will be appreciated and understood by those having ordinary skill in the art. All measurements are made at 25°C, unless otherwise specified.

The present invention generally provides oral care compositions comprising a lactose matrix encapsulate containing at least one care ingredient encapsulated. The present invention specifically provides a composition comprising an encapsulate entrained in a carrier, wherein the encapsulate comprises: at least one oral care ingredient encapsulated in a matrix, wherein the matrix comprises: lactose monohydrate; and a microcrystalline cellulose, wherein the lactose monohydrate is present in an amount of 50% to 75% by weight of the encapsulate, and wherein the oral care ingredient is zinc citrate in an amount of 0.1% to 50% by weight of the encapsulate. The description further provides personal care compositions comprising a lactose matrix encapsulate containing at least one personal care ingredient encapsulated. Preferably, the oral care and/or personal care ingredient is an active ingredient. As referred to herein, an "oral or personal care composition" is any composition that is suitable for administration or application to a human or animal subject for enhancing the health, hygiene or appearance of the subject, including the prevention or treatment of any physiologic condition or disorder, and providing sensory, decorative or cosmetic benefits and combinations thereof. Compositions of the invention are typically oral care compositions. The description further provides skin care compositions, hair care composition, topical pharmaceutical compositions, and ocular compositions. By "oral care composition" as used herein is meant a composition for which the intended use can include oral care, oral hygiene, or oral appearance, or for which the intended method of use can comprise administration to the oral cavity.

In certain embodiments, the encapsulates are beads.

In one embodiment, a dentifrice composition comprises an orally acceptable carrier and lactose encapsulates distributed throughout the carrier. The lactose encapsulates include within their capsule at least one oral care ingredient, preferably an active ingredient. The lactose encapsulates may be present in any suitable amount. In some embodiments, the lactose encapsulates are present in an amount of 0.1% to 20%, 0.5% to 10%, or 1% to 5% by weight, based on the entire weight of the dentifrice composition.

The encapsulates may be of any size. In some embodiments, the average particle size of the encapsulates may be 0.1 mm to 1 mm, 0.3 mm to 0.5 mm, 1 mm to 3.5 mm, or up to 5 mm in diameter.

The encapsulates of the present invention comprise the disaccharide lactose, such as is commonly available as lactose monohydrate. The lactose monohydrate is present in an amount from 50% to 75% by weight of the encapsulate. In the present invention, the encapsulate further comprises a microcrystalline cellulose.

In some embodiments, the encapsulates may include other components. Preferred components include the combination of microcrystalline cellulose and hydroxypropyl methyl cellulose (available commercially as Pharmacoat 606); aesthetic elements, such as titanium dioxide, pigments, dyes, lakes, or other colorants or opacifying agents; and flavor or cooling ingredients, such as menthyl lactate/menthol. The microcrystalline cellulose may be present in an amount from 25% to 40%. The titanium dioxide may be present in an amount from 0.1% to 2%. The pharamacoat 606 may be present in an amount 0.1% to 1%. The methyl lactate/menthol may be present in an amount from 1% to 10%.

In certain embodiments, the lactose monohydrate is present in an amount that is greater than the microcrystalline cellulose. In certain embodiments, the amount of lactose monohydrate is 50 to 75 weight %, and the amount of the microcrystalline cellulose is 25 to 40 weight %.

In certain embodiments, the encapsulate contains a combination of lactose monohydrate and microcrystalline cellulose such that when used in an oral care composition, the encapsulate is capable of dissolving in the oral cavity during the use of the oral care composition to release an ingredient to the oral cavity. During use can include brushing teeth and using a mouthwash.

The description provides other suitable binding agents which may be used, such as ethyl cellulose, starch, acacia gum, guar gum, lecithin, hydroxyalkyl cellulose, polyacrylic acid, crosslinked polyacrylic acid, polyvinyl pyrollidone, and mixtures and copolymers thereof.

The lactose encapsulates may be made in accordance with any known or developed method in the art. Such encapsulates should, however, be capable of releasing an oral care ingredient into the oral cavity upon application. Such release may be effected by any means or mode, including instantaneous or substantially instantaneous release, continuous release, mechanical means, and/or chemical means (such as dissolution).

In various embodiments, the lactose encapsulates may comprise active ingredients. The active ingredients may be incorporated as part of the lactose matrix. In other embodiments, the active ingredients may be encapsulated in the lactose matrix. The active ingredients may be present in an amount from 0.1%-50%, more preferably from 0.5% to 40%, and most preferably from 1% to 30%.

In the present invention, the encapsulate comprises zinc citrate in an amount of 0.1% to 50% by weight of the encapsulate, and optionally, further oral care ingredients.

In various embodiments, the active ingredient may comprise therapeutic actives. As referred to herein, a therapeutic active is a material that is useful for the prevention or treatment of a physiological disorder or condition. Such disorders or conditions include those of the oral cavity (including the teeth and gingiva), skin, hair, and eyes. The specific therapeutic active is preferably determined according to the desired utility of the composition. Such actives include, but are not limited to, the following.
A. antimicrobial agents, such as triclosan, cetyl pyridium chloride, domiphen bromide, quaternary ammonium salts, sanguinarine, fluorides, alexidine, octonidine, EDTA, essential oils such as thymol, methyl salicylate, eucalyptol and menthol, and zinc compounds,
B. non-steroidal anti-inflammatory drugs, such as aspirin, acetaminophen, ibuprofen, ketoprofen, diflunisal, fenoprofen calcium, naproxen, tolmetin sodium, indomethacin.
C. anti-tussives, such as benzonatate, caramiphen edisylate, menthol, dextromethorphan hydrobromide, chlophedianol hydrochloride,
D. decongestants, such as pseudoephedrine hydrochloride, phenylepherine, phenylpropanolamine, pseudoephedrine sulfate,
E. anti-histamines, such as brompheniramine maleate, chlorpheniramine maleate, carbinoxamine maleate, clemastine fumarate, dexchlorpheniramine maleate, diphenhydramine hydrochloride, diphenylpyraline hydrochloride, azatadine meleate, diphenhydramine citrate, doxylamine succinate, promethazine hydrochloride, pyrilamine maleate, tripelennamine citrate, triprolidine hydrochloride, acrivastine, loratadine, brompheniramine, dexbrompheniramine,
F. expectorants, such as guaifenesin, ipecac, potassium iodide, terpin hydrate, and the like,
G. anti-diarrheals, such a loperamide,
H. H₂ -antagonists, such as famotidine, ranitidine; and
I. proton pump inhibitors, such as omeprazole, lansoprazole,
J. general nonselective CNS depressants, such as aliphatic alcohols, barbiturates,
K. general nonselective CNS stimulants such as caffeine, nicotine, strychnine, picrotoxin, pentylenetetrazol,
L. drugs that selectively modify CNS function such as phenyhydantoin, phenobarbital, primidone, carbamazepine, ethosuximide, methsuximide, phensuximide, trimethadione, diazepam, benzodiazepines, phenacemide, pheneturide, acetazolamide, sulthiame, bromide,
M. antiparkinsonism drugs such as levodopa, amantadine,
N. narcotic-analgesics such as morphine, heroin, hydromorphone, metopon, oxymorphone, levorphanol, codeine, hydrocodone, xycodone, nalorphine, naloxone, naltrexone,
O. analgesic-antipyretics such as salycilates, phenylbutazone, indomethacin, phenacetin,
P. psychopharmacological drugs such as chlorpromazine, methotrimeprazine, haloperidol, clozapine, reserpine, imipramine, tranylcypromine, phenelzine, lithium,
Q. L-arginine.

The amount of the therapeutic active that can be used in the encapsulates can be dependent upon the dose needed to provide an effective amount of the medicament. Preferred therapeutic actives include zinc compounds, cetylpyridinium chloride, and L-arginine. Those skilled in the art will be capable of determining the appropriate amount of active to include in the inventive encapsulates, using the guidelines provided herein.

In certain oral care embodiments, the encapsulate may comprise an oral care active, which is useful for the prevention or treatment of an oral care disorder or condition. Oral care actives among those useful herein include abrasives, anticaries agents, tartar control agents, antiplaque agents, periodontal actives, breath freshening agents, flavorants, colorants, malodour control agents, tooth desensitizers, salivary stimulants, whitening agents, and combinations thereof. Active materials among those useful herein are described in U.S. Patent 6,596,298, Leung et al.,.

Tartar control agents among those useful herein include dialkali or tetraalkali metal pyrophosphate salts such as Na₄P₂O₇, K₄P₂O₇, Na₂K₂P₂O₇, Na₂H₂P₂O₇ and K₂H₂P₂O₇; long chain polyphosphates such as sodium hexametaphosphate; and cyclic phosphates such as sodium trimetaphosphate. In some configurations, a polyphosphate is a beta.-phase calcium pyrophosphate, such as disclosed in US Patent 6,241,974White, Jr..

Odor reducing agents useful herein include sulfur precipitating agents. Such sulfur-precipitating agents include metal salts, such as a copper salt or a zinc salt. Such salts include copper gluconate, zinc citrate and zinc gluconate.

The encapsulates of certain embodiments include a zinc compound that provides a source of zinc ions. The zinc compound can be a soluble or sparingly soluble compound of zinc with inorganic or organic counter ions. Examples include the fluoride, chloride, chlorofluoride, acetate, hexafluorozirconate, sulfate, tartrate, gluconate, citrate, lactate, malate, glycinate, pyrophosphate, metaphosphate, oxalate, phosphate, carbonate salts, and oxides of zinc. Examples of suitable zinc compounds that serve as zinc ion sources are zinc oxide, zinc sulfate, zinc chloride, zinc citrate, zinc lactate, zinc gluconate, zinc malate, zinc tartrate, zinc carbonate, zinc phosphate, and other salts listed in U.S. Pat. No. 4,022,880.

In the present invention, the encapsulate comprises zinc citrate in an amount of 0.1% to 50% by weight of the encapsulate, and optionally, further oral care ingredients.

In certain embodiments, the encapsulates include a fluoride compound that provides a source of fluoride ions. The fluoride ion source herein may be a soluble fluoride source capable of providing free fluoride ions. Soluble fluoride ion sources include sodium fluoride, stannous fluoride, indium fluoride, zinc fluoride, and sodium monofluorophosphate. Norris et al., U.S. Pat. No. 2,946,725, issued Jul. 26, 1960, and Widder et al., U.S. Pat. No. 3,678,154 issued Jul. 18, 1972disclose such fluoride ion sources as well as others. Stannous fluoride are preferred soluble fluoride ion sources.

In certain embodiments, the encapsulate may include a saliva stimulating agent (a "succulent"). Such agents include those disclosed in U.S. Pat. No. 4,820,506, Kleinberg et al. In some configurations, a saliva stimulating agent can include a food acid such as citric, lactic, malic, succinic, ascorbic, adipic, fumaric and tartaric acids. In some embodiments, a saliva stimulating agent can be used in the amelioration of dry mouth.

In certain oral care embodiments, the encapsulate comprises other active materials, such as antibacterial agents including magnolia extracts, triclosan, grapeseed extracts, thymol, methyl salicylate, eucalyptol, menthol, hop acids, cetyl pyridinium chloride, (including CPC/Zn and CPC + enzymes) and usnic acid; anti-inflammatory agents such as breath freshening agents (for example zinc gluconate, zinc citrate, zinc chlorite and alpha ionone); tooth desensitizers such as potassium nitrate, desensitizing polymers, and desensitizing minerals; anti-inflammatory agents such as magnolia extract, ursolic acid; aloe, and cranberry extract; vitamins such as pantheon, retinyl palmitate, folic acid, tocopherol acetate and Vitamin A; herbs or herbal extracts such as rosemary, oregano, chamomilla recutita, mentha piperita, salvia officinalis, orcommiphora and myrrha; proteins, such as milk proteins and enzymes such as peroxide-producing enzymes, amylase, plaque-disrupting agents such as papain, glucoamylase, glucose oxidase, and "next generation" enzymes; whitening agents such as hydrogen peroxide, urea peroxide and phosphate salts; medicinals, such as aspirin (acetyl salicylic acid), caffeine, and benzocaine; probiotics; abrasives such as silicas (including high cleaning silica); anti-caries agents such as stannous salts (e.g., stannous fluoride) or amino fluoride; a nitric oxide synthase inhibitor such as guanidinoethyldisulfide; calcium; antiattachmetn ingredients, such as polyumylphosphonic acid; preservatives such as Solbrol® (Bayer Chemicals AG);silicones; chlorophyll compounds, anti-leukoplakia agents such as beta-carotene; anti-oxidants such as Vitamin E; and combinations thereof. In some embodiments, the encapsulates comprise such active materials at a concentration of 0.01 to 30% by weight of encapsulate, from 2% to 25% by weight of the encapsulate, or from 10% to 20% by weight of encapsulate.

In various embodiments, the encapsulate comprises a formulation colorant that imparts a color to the encapsulate, the composition, or both. In various embodiments, the encapsulates contrast with the carrier, and are white, black, or of any color that is visible against or contrasts with the carrier background. Formulation colorants among those useful herein include non-toxic water soluble dyes or pigment, such as, for example, metallic oxide "lakes." In certain embodiments, the colorant is approved for incorporation into a food or drug by a regulatory agency, such as FD&C or D&C pigments and dyes approved by the FDA for use in the United States. Colorants among those useful herein include FD&C Red No. 3 (sodium salt of tetraiodofluorescein), Food Red 17, disodium salt of 6-hydroxy-5-{(2-methoxy-5-methyl-4-sulphophenyl)azo}-2-naphthalenesulfonic acid, Food Yellow 13, sodium salt of a mixture of the mono and disulphonic acids of quinophtalone or 2-(2-quinolyl) indanedione, FD&C Yellow No. 5 (sodium salt of 4-p-sulfophenylazo-1-p-sulfophenyl-5-hydroxypyrazole-3 carboxylic acid), FD&C Yellow No. 6 (sodium salt of p-sulfophenylazo-B-naphtol-6-monosulfonate), FD&C Green No. 3 (disodium salt of 4-{[4-(N-ethyl-p-sulfobenzylamino)-phenyl]-(4-hydroxy-2-sulfoniumphenyl)-methylene}-[1-(N-ethyl-N-p-sulfobenzyl)-Δ-3,5-cyclohexadienimine], FD&C Blue No. 1 (disodium salt of dibenzyldiethyl-diaminotriphenylcarbinol trisulfonic acid anhydrite), FD&C Blue No. 2(sodium salt of disulfonic acid of indigotin), and mixtures thereof in various proportions. In one embodiment, the colorant comprises a water insoluble inorganic pigment, such as titanium dioxide, chromium oxide green, phthalocyanine green, ultramarine blue, ferric oxide, or a water insoluble dye lake. In some embodiments, dye lakes include calcium or aluminum salts of an FD&C dye such as FD&C Green #1 lake, FD&C Blue #2 lake, D&C Red #30 lake or FD&C # Yellow 15 lake. In certain embodiments, a water soluble dye, such as, for example, FD&C Blue #1, is contained within a water-insoluble polymer such as, for example polyethylene such as that found in polyethylene encapsulates (e.g., Microblue Spectrabeads, sold by Micropowders, Inc.). In certain embodiments, the encapsulate comprises a dye such as D&C Red #30. In certain embodiments, a white colorant is used, for example titanium dioxide (TiO₂), titanium dioxide coated mica (e.g., Timiron), a mineral, or a clay. In certain embodiments, the colorant is a non-bleeding dye. In various embodiments, the encapsulate comprises a colorant at a level of from 0.5% to 20% by weight of the encapsulate, or from 1% to 15% by weight of the encapsulate, or from 3% to 12% by weight of the encapsulate. In one embodiment, the compositions of the present invention comprise a first plurality of encapsulates comprising a first color, and a second plurality of encapsulates comprising a second color. Preferably, the second color is different than the first color, (including, for example, of a different tint strength, or of a different hue).

In certain embodiments, the entrainment of the active ingredients in the encapsulate matrix suspended in the dentifrice or other composition isolates these agents from interaction with reactive ingredients present in the composition so that the agents are maintained substantially separate from the reactive composition ingredients during manufacture and storage while subsequently being released from the encapsulate matrix when the composition is used. Isolation not only avoids adverse reactions that may occur between the therapeutic actives and other components that are present in the carrier material, but also avoids dissolution of the therapeutic actives and premature release of actives.

The compositions of the present invention comprise a carrier in which an encapsulate is entrained. As referred to herein, a "carrier" is any material or composition in which an encapsulate can be entrained and is suitable for administration or application to the human or animal subject to whom the composition is administered or applied. As referred to herein, "entrained" refers to the embedding or suspension of an encapsulate in a carrier. In various embodiments comprising a plurality of encapsulates, such encapsulates may be entrained by embedding, suspension, dispersion or other distribution of the encapsulates in the carrier. In various embodiments, the encapsulates are distributed substantially homogenously throughout the carrier. In other embodiments, the encapsulates are not distributed homogenously in the carrier. In certain embodiments, the distribution of a plurality of encapsulates is substantially isotropic within the carrier.

The compositions of the embodiments may be described as comprising two phases, wherein one phase comprises a carrier and a second phase comprises the aforementioned encapsulate. The term "phase" as used herein denotes a physical phase as understood in the physical and material sciences, i.e., a portion of a material whose properties and composition are uniform. However, a phase as used herein can be discontinuous, i.e., a phase can comprise a plurality of separate components. For example, a plurality of polymer encapsulates of identical composition is considered to comprise a single phase. In some embodiments, an encapsulate can be entirely embedded within the material comprising the first phase, or totally or partially exposed on the surface of the first phase. For example, if the composition is a dentifrice comprising both a gel and encapsulates, an encapsulate can be totally surrounded by the gel, or partially or totally exposed on the surface of the gel. In certain embodiments, compositions comprise more than two phases. Such multi-phase compositions include those having two carriers, each of which contributes a phase to the composition, in addition to encapsulates as described herein. Other multi-phase compositions include those having a single carrier and two or more pluralities of encapsulates, wherein the pluralities of encapsulates have differing compositions.

In various embodiments, the carrier is a liquid, semi-solid or solid. A "liquid" can be a liquid of low or high viscosity. A liquid can be a liquid such that flow is imperceptible under ambient conditions. For example, a soap, such as an ordinary bar of hand soap, can be considered a liquid herein. A liquid can be a thixotropic liquid. A "semi-solid" as used herein can be a gel, a colloid, or a gum. As used herein, semi-solids and liquids are fluids distinguished on the basis of viscosity: a semi-solid is a high viscosity fluid, while a liquid has lower viscosity. There is no definitive dividing line between these two types of fluids. A semi-solid can, in certain embodiments, have a viscosity as high as thousands of mPa·s. Carriers among those useful herein include liquids, pastes, ointments, and gels, and can be transparent, translucent or opaque.

In certain embodiments, the compositions of the present invention are oral care compositions, suitable for administration to the oral cavity. Such compositions include dentifrices, mouthwashes, dental gels, lozenges, beads, gums, oral strips, mints, liquid toothpastes, sprays, paint-on gels, lip balms, whitening strips, breath strips, oral chews, powders, foams, and combinations thereof. An oral care composition disclosed herein can be used, for example, for cavity prevention, whitening, plaque prevention or reduction, gingivitis prevention or reduction, tartar control, sensitivity prevention or reduction, or breath malodor prevention or reduction, and stain prevention.

The specific composition of the carrier preferably depends on the intended use of the composition. In various embodiments, the carrier is aqueous, comprising from 5% to 95% water or from 10% to 70% water. In other embodiments, the carrier is substantially non-aqueous. In a dentifrice carrier, water content can be from 5% to 70%, from 10% to 50%, or from 20% to 40%. When the presence of water will cause the encapsulate to disintegrate, it is particularly preferred that the dried encapsulate contain no free water, in which the amount of water is substantially 0%, or negligible.

The carrier may comprise any of a variety of materials, including emulsifiers, thickeners, fillers, and preservatives. In some embodiments, the carrier may include a functional or active material, such as those described above. In some embodiments, the carrier comprises the same functional material as the encapsulate.

In some embodiments, the carrier is suitable for use as a dentifrice. In some embodiments, the carrier comprises a humectant, such as glycerine, sorbitol or an alkylene glycol such as polyethylene glycol or propylene glycol. Carrier compositions among those useful herein are disclosed in U.S. Patents 5,695,746, Garlick, Jr., et al*,* and 4,839,157, Mei-King Ng et al.

In certain dentifrices, the carrier comprises additional surfactants or mixture of surfactants. Surfactants among those useful herein include water-soluble salts of at least one higher fatty acid monoglyceride monosulfate, such as the sodium salt of the monsulfated monoglyceride of hydrogenated coconut oil fatty acids; cocamidopropyl betaine; a higher alkyl sulfate such as sodium lauryl sulfate; an alkyl aryl sulfonate such as sodium dodecyl benzene sulfonate; a higher alkyl sulfoacetate; sodium lauryl sulfoacetate; a higher fatty acid ester of 1,2-dihydroxy propane sulfonate; and a substantially saturated higher aliphatic acyl amides of a lower aliphatic amino carboxylic acid, such as those having 12 to 16 carbons in the fatty acid, alkyl or acyl radicals; and mixtures thereof. Amides can be, for example, N-lauroyl sarcosine, and the sodium, potassium, and ethanolamine salts of N-lauroyl, N-myristoyl, or N-palmitoyl sarcosine. In various embodiments the carrier comprises the surfactant at a level of from 0.3% to 5% by weight of composition, or 0.5% to 3% by weight of composition.

In certain embodiments, organic solvents or polymers include ethylene oxide-based or propylene oxide-based non-ionic polyethers such as polyethylene glycol (PEG), polyethylene oxide (PEO), polypropylene oxide (PPO), polypropylene glycol (PPG) and co-polymers comprised of any combination of these polymers.

In various dentifrice embodiments, the carrier comprises thickeners, gelling agents or combinations thereof. Thickeners or gelling agents useful herein include inorganic, natural or synthetic thickeners or gelling agents. Examples of thickeners and gelling agents useful herein include inorganic thickening silicas such as: an amorphous silica, for example Zeodent® 165 (Huber Corporation); Irish moss; iota-carrageenan; gum tragacanth; or polyvinylpyrrolidone. In certain embodiments, the carrier comprises a polishing agent, such as a silica, a calcined alumina, sodium bicarbonate, calcium carbonate, dicalcium phosphate or calcium pyrophosphate. In various embodiments, the carrier can be a visually clear composition.

In certain embodiments, the oral care composition includes a preservative. A preservative can be added in amounts from about 0.001 wt % to about 5 wt %, preferably from about 0.01 wt % to about 1 wt % of the encapsulate. Non-limiting examples of preservatives include sodium benzoate and potassium sorbate.

In various dentifrice embodiments, comprising a visually clear carrier, the composition comprises at least one polishing agent. Polishing agents among those useful herein include collodial silica, such as, for example, Zeodent® 115 (Huber Corporation), and alkali metal aluminosilicate complexes (i.e., a silica comprising alumina). In some configurations, a polishing agent can have a refractive index close to that of a gelling agent combined with water and/or humectant.

In certain embodiments, a composition can comprise, in addition to encapsulates as described herein, two or more carriers, each of which contributes a phase to the composition. Such a composition can be stable to color bleeding.

The compositions of the present invention are preferably stable under normal conditions of storage. As referred to herein, "stable" refers to the lack of significant adverse effect on one, and preferably all, compositional attributes such as appearance, flavor, rheology, and chemical composition of the composition. Preferably, stability in the present compositions includes the compositional and physical stability of encapsulate in the composition. In various embodiments a composition comprising an encapsulate is stable upon storage at ambient temperature for at least about two years. It is understood, however, that in some embodiments, an otherwise stable encapsulate can disintegrate during use (as discussed above), for example, during toothbrushing using a dentifrice composition.

The description also provides processes for making compositions comprising lactose matrix encapsulates entrained in a carrier. In various embodiments, a plurality of encapsulates are combined with a carrier. In some configurations, a carrier and a plurality of encapsulates can be mixed. In some configurations, the mixing can comprise slow stirring. In one preferred embodiment, the process for making the composition including a carrier having distributed therein a plurality of lactose matrix encapsulates includes:
(a) providing the carrier;
(b) adding encapsulates containing at least one oral care and/or personal care ingredient to the carrier to form a mixture; and
(c) homogenizing the mixture.

The term "homogenizing" as used herein refers to the admixture of the encapsulates and the carrier so as to attain a substantially homogeneous distribution of encapsulates in the carrier. It should be noted, however, that the resulting composition still retains two-phase composition characteristics. Homogenizing may be accomplished using any of a variety of conventional homegenizers.

In another method, the encapsulate is added to a component of the carrier (e.g., to a humectant for a dentifrice). The remainder of the carrier then may be made, and the mixture of encapsulate then added to the carrier.

In various aspects, the present description provides methods for administering a functional material to a human or animal subject in need thereof, comprising topically applying to said subject a composition comprising a lactose matrix encapsulate entrained in a carrier, wherein the encapsulate includes at least one oral care and/or personal care ingredient, preferably an active ingredient. As referred to herein, "administering" refers to any method by which a composition is applied on or administered to the subject. In various embodiments, the administration is topical, wherein the composition is applied to an external surface of the subject, such as to a surface of the oral cavity (e.g., teeth, gingival, and tongue). The specific route and method of administration will depend, of course, on the intended use of the composition.

In oneaspect, the method additionally comprises disrupting the encapsulate after topically applying the encapsulate. Such disruption may be accomplished by any of a variety of methods, including chemical and/or mechanical means. Chemical means include degradation of the encapsulate by contact with water or a material present at the site of administration (e.g., saliva in an oral care application). Physical means include agitation, grinding, and shear forces produced by application of physical energy to the composition during use (e.g., brushing in a dentifrice application).

In various aspects, the description provides methods for the treatment of an oral care condition. As referred to herein, an "oral care condition" is any disorder or condition which can be prevented or treated by administration of a composition to the oral cavity, including disorders or conditions of the teeth, oral mucosa, gingiva and tongue. Such conditions include caries, gingivitis, periodontitis, and cosmetic conditions such as yellowing and malodour.

The embodiments described herein can be further understood by reference to the following non-limiting examples.

### SPECIFIC EMBODIMENTS OF THE INVENTION

### Encapsulate Composition

This example illustrates an encapsulate according to an embodiment of the invention.

| Material | Weight % |
|---|---|
| Lactose Monohydrate | 50-75 |
| Microcrystalline Cellulose | 25-40 |
| Zinc Citrate Trihydrate | 0.1-50 |
| Titanium Dioxide | 0.1-2 |
| Pharmacoat 606 hydroxypropyl methyl cellulose | 0.1-1 |
| Menthyl Lactate/Menthol | 1-10 |

### Example 1

This example illustrates a typical formulation according to an embodiment of the invention.

**Table 1: Formulation with Zinc Citrate in a Lactose Matrix Encapsulate**

| **Ingredient** | **% wt/wt** |
|---|---|
| Deionized Water | 10.149 |
| Sodium Fluoride | 0.243 |
| Polyethylene Glycol 600 | 3.000 |
| Tetrasodium Pyrophosphate | 0.500 |
| Sorbitol | 56.438 |
| Lactose Encapsulates with Zinc Citrate | 1.500 |
| Sodium CMC 2000S | 0.650 |
| Sodium Saccharin | 0.350 |
| Zeodent 105 silica | 10.000 |
| Zeodent 114 silica | 10.000 |
| Zeodent 165 silica | 2.750 |
| Red No. 33, 1% Solution | 0.110 |
| Blue Color Solution | 0.160 |
| Flavor | 1.400 |
| Sodium Lauryl Sulfate | 1.500 |
| Betaine | 1.250 |

### Example 2

This example illustrates the effectiveness of a typical formulation according to an embodiment of the invention in controlling volatile sulfur compounds as compared to commercial products. Zinc salts have been widely used in oral compositions for oral malodor control. Mouth odor is believed to be caused by the metabolic pathways of bacteria in the oral cavity. Volatile sulfur compounds (VSC) such as hydrogen sulfide and methylmercaptan, make up 85% of bad breath. Zinc citrate fights bad breath by inhibiting the metabolism of bacteria and the conversion of VSCs into malodorous compounds and volatile sulfides.

An *in-vitro* study was conducted to measure the reduction of VSC. Four formulations were used in the study. The positive control was a commercially available 0.5% zinc strip formulation sold under the name MaxFresh® Night. The negative control was a MaxFresh® Night formulation mouthwash encapsulates replacing the zinc strip. Two prototype formulations using lactose encapsulates were made by replacing the zinc strip in the MaxFresh® Night formulation with lactose encapsulates comprising an active ingredient. One prototype formulation comprised zinc oxide lactose encapsulates and the other prototype formulation comprised zinc citrate lactose encapsulates.

The results of the *in-vitro* study were as follows: the negative control produced an 18.33% VSC reduction, the positive control produced a 26.13% VSC reduction, the zinc oxide lactose encapsulate prototype formulation produced a 16.4% VSC reduction, and the zinc citrate lactose encapsulate prototype formulation produced a 25.12% VSC reduction.

The prototype formula with zinc citrate encapsulates performed similarly to the positive control, the MaxFresh® Night formula with zinc strip. The MaxFresh® Night formula with zinc strip has shown efficacy clinically.

### Example 3

The two prototype formulas described in example 2 were evaluated for zinc astringency versus the MaxFresh® Night product containing zinc strips. Based on evaluation by trained sensory experts, the zinc encapsulate formulas had an overall improvement on taste versus the current MaxFresh® Night formula with zinc strips. One hypothesis is that the improved taste is due to the removal of film strips. As previously discussed, zinc salts have been well used in dentistry for controlling bad breath. However, the downside of having zinc ions in oral compositions is the astringent taste. The mouth feel is described as puckering, and the zinc is also associated with a sour or bitter taste. This flavor test has shown that lactose encapsulates containing zinc compounds improve the taste of compositions comprising zinc compounds.

The examples and other embodiments described herein are exemplary and not intended to be limiting in describing the full scope of compositions and methods of this invention.

## Claims

1. A composition comprising an encapsulate entrained in a carrier, wherein the encapsulate comprises:
at least one oral care ingredient encapsulated in a matrix,
wherein the matrix comprises:
lactose monohydrate; and
a microcrystalline cellulose, wherein the lactose monohydrate is present in an amount of 50% to 75% by weight, of the encapsulate, and wherein the oral care ingredient is zinc citrate in an amount of 0.1% to 50% by weight of the encapsulate.

2. The composition of claim 1, wherein the encapsulate further comprises a coloring agent.

3. The composition of claim 2, wherein the coloring agent comprises at least one coloring agent chosen from titanium dioxide, a dye, a pigment, a colorant, or an opacifying agent.

4. The composition of claim 1, wherein the microcrystalline cellulose is present in an amount of 25% to 40% by weight of the encapsulate.

5. The composition of claim 3, wherein the titanium dioxide is present in an amount of 0.1% to 2% by weight of the encapsulate.

6. The composition of any preceding claim, wherein the encapsulate has a size of 0.5 mm to 5 mm in diameter.

7. The composition of any preceding claim, wherein the encapsulate contains a combination of lactose monohydrate and microcrystalline cellulose such that when used in an oral care composition, the encapsulate is capable of dissolving in the oral cavity during the use of the oral care composition to release an ingredient to the oral cavity.

8. The composition of any preceding claim, where in the carrier is an orally acceptable carrier, and the composition is an oral care composition.

9. The composition of any preceding claim, wherein the encapsulate is present in an amount of 0.1% to 20% by weight.

10. A composition according to claims 1 to 9 for use in a method of providing oral health benefits to an oral surface, wherein the use comprises contacting the composition with the oral surface.

11. A composition for use according to claim 10, wherein the use comprises disrupting the encapsulates after topical application.

## Patentansprüche

1. Zusammensetzung umfassend eine in einen Träger eingetragene Kapsel, worin die Kapsel umfasst:
mindestens ein Mundpflegebestandteil eingekapselt in einer Matrix, worin die Matrix umfasst:
Laktosemonohydrat und eine mikrokristalline Cellulose, worin das Laktosemonohydrat in einer Menge von 50 - 75 Gewichtsprozent der Kapsel vorliegt und worin der Mundpflegebestandteil Zinkzitrat in einer Menge von 0,1 bis 50 Gewischtsprozent der Kapsel ist.

2. Zusammensetzung nach Anspruch 1, worin die Kapsel weiterhin ein Farbmittel umfasst.

3. Zusammensetzung nach Anspruch 2, worin das Farbmittel mindestens ein Färbungsmittel umfasst ausgewählt aus Titandioxid, einem Farbstoff, einem Pigment, einem Färbungsmittel oder einem Trübungsmittel.

4. Zusammensetzung nach Anspruch 1, worin die mikrokristalline Cellulose in einer Menge von 25 bis 40 Gewichtsprozent der Kapsel vorliegt.

5. Zusammensetzung nach Anspruch 3, worin das Titandioxid in einer Menge von 0,1 bis 2 Gewichtsprozent der Kapsel vorliegt.

6. Zusammensetzung nach irgendeinem vorhergehenden Anspruch, worin die Kapsel eine Größe von 0,5 mm bis 5 mm im Durchmesser hat.

7. Zusammensetzung nach irgendeinem vorhergehenden Anspruch, worin die Kapsel eine Kombination aus Laktosemonohydrat und mikrokristalline Cellulose enthält, so dass bei der Verwendung in einer Mundpflegezusammensetzung die Kapsel in der Lage ist in der Mundhöhle sich während der Verwendung der Mundpflegezusammensetzung aufzulösen, um einen Bestandteil in die Mundhöhle abzugeben.

8. Zusammensetzung nach irgendeinem vorhergehenden Anspruch, wobei der Träger ein oral annehmbarer Träger ist und die Zusammensetzung eine Mundpflegezusammensetzung ist.

9. Zusammensetzung nach irgendeinem vorhergehenden Anspruch, wobei die Kapsel in einer Menge von 0,1 bis 20 Gewichtsprozent vorliegt.

10. Zusammensetzung nach den Ansprüchen 1 bis 9 zur Verwendung in einem Verfahren zur Bereitstellung von Vorteilen für die orale Gesundheit an eine orale Oberfläche, wobei die Verwendung das Inkontaktbringen der Zusammensetzung mit der oralen Oberfläche umfasst.

11. Zusammensetzung zur Verwendung nach Anspruch 10, wobei die Verwendung das Aufbrechen der Kapseln nach topischer Anwendung umfasst.

## Revendications

1. Composition comprenant un produit encapsulé entraîné dans un support, dans laquelle le produit encapsulé comprend :
au moins un ingrédient de soin buccal encapsulé dans une matrice,
dans laquelle la matrice comprend :
du lactose monohydraté ; et
une cellulose microcristalline, dans laquelle le lactose monohydraté est présent en une quantité de 50 % à 75 % en poids, du produit encapsulé, et dans laquelle l'ingrédient de soin buccal est du citrate de zinc en une quantité de 0,1 % à 50 % en poids du produit encapsulé.

2. Composition selon la revendication 1, dans laquelle le produit encapsulé comprend en outre un agent colorant.

3. Composition selon la revendication 2, dans laquelle l'agent colorant comprend au moins un agent colorant choisi parmi le dioxyde de titane, un agent teintant, un pigment, un colorant ou un agent opacifiant.

4. Composition selon la revendication 1, dans laquelle la cellulose microcristalline est présente en une quantité de 25 % à 40 % en poids du produit encapsulé.

5. Composition selon la revendication 3, dans laquelle le dioxyde de titane est présent en une quantité de 0,1 % à 2 % en poids du produit encapsulé.

6. Composition selon l'une quelconque des revendications précédentes, dans laquelle le produit encapsulé a une taille de 0,5 mm à 5 mm de diamètre.

7. Composition selon l'une quelconque des revendications précédentes, dans laquelle le produit encapsulé contient une combinaison de lactose monohydraté et de cellulose microcristalline de sorte que lorsqu'il est utilisé dans une composition de soin buccal, le produit encapsulé est apte à se dissoudre dans la cavité buccale pendant l'utilisation de la composition de soin buccal pour libérer un ingrédient dans la cavité buccale.

8. Composition selon l'une quelconque des revendications précédentes, où le support est un support acceptable par voie orale, et la composition est une composition de soin buccal.

9. Composition selon l'une quelconque des revendications précédentes, dans laquelle le produit encapsulé est présent en une quantité de 0,1 % à 20 % en poids.

10. Composition selon les revendications 1 à 9, pour une utilisation dans un procédé de fourniture d'avantages pour la santé buccale à une surface buccale, dans laquelle l'utilisation comprend la mise en contact de la composition avec la surface buccale.

11. Composition pour une utilisation selon la revendication 10, dans laquelle l'utilisation comprend la rupture des produits encapsulés après application topique.
